# FASCICULE DE BREVET EUROPEEN

(11) **EP 1 071 692 B1**
(45) Date de publication et mention de la délivrance du brevet: **31.07.2002**
(21) Numéro de dépôt: 99915798.5
(22) Date de dépôt: 13.04.1999
(51) Int. Cl.: C07H 3/06, C07H 17/04

(54) **PROCEDE DE SYNTHESE DU LAMINARIBIOSE**
VERFAHREN ZUR SYNTHESE VON LAMINARIBIOSE
METHOD FOR LAMINARIBIOSE SYNTHESIS

(30) Priorité: 14.04.1998 FR 9804610
(43) Date de publication de la demande: 31.01.2001
(73) Titulaire: LABORATOIRES GOEMAR, 35413 Saint-Malo (FR)
(72) Inventeur: YVIN, Jean-Claude, F-35400 Saint Malo (FR); JAMOIS, Frank, F-35000 Rennes (FR); FERRIERES, Vincent, F-35140 SAINT AUBIN DU CORNIER (FR); PLUSQUELLEC, Daniel, F-35230 Noyal Châtillon sur Seiche (FR)
(74) Mandataire: Hubert, Philippe
(86) Numéro de dépôt international: FR9900857
(87) Numéro de publication internationale: WO9952920

(56) Documents cités:
- K.TAKEO: "Synthesis of Benzyl alpha- and beta-Sophorosides, and of benzyl alpha-laminarabioside." CARBOHYDRATE RESEARCH., vol. 86, no. 1, 1980, pages 151-157, XP002090785 AMSTERDAM NL cité dans la demande
- K.TAKEO: "A Convenient Synthesis of Laminarabiose and some of its Glycosides." CARBOHYDRATE RESEARCH., vol. 77, 1979, pages 245-251, XP002090786 AMSTERDAM NL cité dans la demande
- C.FORZATO ET AL.: "Baker's Yeast Reduction of 4-Hetero-2-(2-nitroethyl)cyclohexanones." TETRAHEDRON: ASYMMETRY., vol. 8, no. 11, 1997, pages 1811-1820, XP002090787 OXFORD GB

## Description

La présénte invention concerne un nouveau procédé de préparation par voie chimique du β-D-glucopyranosyl-(1→3)-D-glucopyranose de formule (I), communément appelé Laminaribiose.

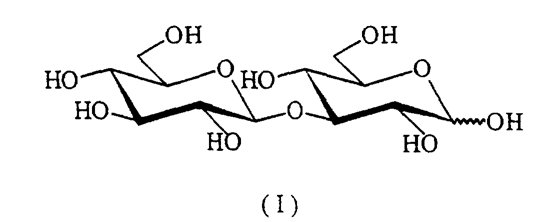

Le Laminaribiose est un disaccharide notamment utilisé dans le domaine agricole et en tant qu'antiseptique.

Ce disaccharide est généralement obtenu par hydrolyse ou acétolyse de polysaccharides naturels d'origine végétale (voir Villa, Phaff, Notario, *Carbohydr. Res.,* 1979, 74, 369 ; Kusama, Kusakabe, Zama, Murakami, Yasui, *Agric*. *Biol. Chem.,* 1984, 48, 1433 ; Wang, Sakairi, Kuzuhara, *Carbohydr*. *Res.,* 1991, 219, 133 ; Moreau, Viladot, Samain, Planas, Driguez, *Bioorg*. *Med*. *Chem.,* 1996, 4, 1849).

Le Laminaribiose peut également être préparé par voie chimique, notamment par des méthodes dérivées de la méthode de *O*-glycosylation de Koenigs-Knorr (voir Koenigs, Knorr, *Ber*. *Dtsch*. *Chem. Ges.,* 1901, 34, 957) utilisant des halogénures de glycosyle comme donneurs de glycosyle.

Une première méthode a ainsi été proposée par Freudenberg et von Oertzen en 1951 (voir Freudenberg, von Oertzen, *Justus Liebigs Ann*. *Chem.,* 1951, 574, 37), et une seconde méthode a été décrite par Bächli et Percival en 1952 (voir Bächli, Percival, *J. Chem. Soc.,* 1952, 1243).

Les inconvénients majeurs de ces deux méthodes résident dans une purification difficile à mettre en oeuvre et dans un rendement global inférieur à 10%.

Une troisième méthode a été proposée par Takeo en 1979 (voir Takeo, *Carbohydr*. *Res*., 1979, 77, 245) mais elle nécessite plusieurs étapes de protection et déprotection sélective des hydroxyles de l'accepteur utilisé qui se présente sous forme glucopyranosique.

On a également proposé de préparer le Laminaribiose à partir d'orthoesters (voir Kochetkov, Bochtov, Sokolovskaya, Snyatkova, *Carbohydr*. *Res*., 1971, 16, 17). Cette méthode s'avère toutefois difficile à mettre en oeuvre et ne permet d'obtenir le Laminaribiose qu'avec un rendement global voisin de 10 %.

Dans ces conditions, la présente invention a pour but de fournir un nouveau procédé de préparation par voie chimique du Laminaribiose présentant un nombre limité d'étapes, d'une mise en oeuvre aisée ET permettant d'obtenir le produit recherché sous forme pure avec un rendement global élevé.

La solution conforme à la présente invention pour résoudre ce problème technique consiste en un procédé de préparation du Laminaribiose comprenant une étape de couplage glycosidique entre un donneur et un accepteur de glycosyle, caractérisé en ce que :
- le donneur de glycosyle se présente sous forme pyranosique et répond à la formule (II) 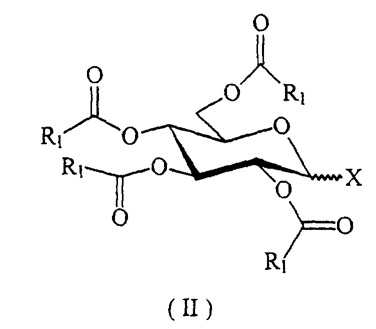 dans laquelle :
   - R₁: représente :
   - un radical alkyle ou halogénoalkyle ayant de 1 à 6 atomes de carbone ;
   - un radical aryle non substitué ou substitué par un ou plusieurs groupements choisis parmi un atome d'halogène, un radical alcoxy ayant de 1 à 6 atomes de carbone ou un groupement nitro ;
   - X: représente un groupe partant nucléofuge choisi parmi :
   - un groupement de formule S(O)ₙR' dans laquelle R' représente un radical alkyle ayant de 1 à 6 atomes de carbone, un radical aryle non substitué ou substitué par un groupement alcoxy ayant de 1 à 6 atomes de carbone, un groupement nitro ou acétamide, et n est un nombre entier égal à 0 ou 1 ;
   - un groupement trichloroacétimidate
- l'accepteur de glycosyle se présente sous forme furanosique et répond à la formule (III) 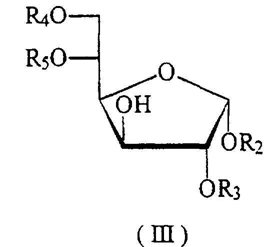 dans laquelle :
   - R₂ et R₃: forment ensemble un radical méthylidyle, éthylidyle, trichloroéthylidyle, isopropylidyle, hexafluoroisopropylidyle, cyclopentylidyle, cyclohexylidyle, cycloheptylidyle, butylidyle, 1-tertiobutyléthylidyle, 1-phényléthylidyle, benzylidyle, méthoxybenzylidyle, 1-phénylbenzylidyle ;
   - R₄ et R₅: forment ensemble un radical méthylidyle, éthylidyle, trichloroéthylidyle, isopropylidyle, hexafluoroisopropylidyle, cyclopentylidyle, cyclohexylidyle, cycloheptylidyle, butylidyle, 1-tertiobutyléthylidyle, 1-phényléthylidyle, benzylidyle, méthoxybenzylidyle, 1-phénylbenzylidyle ou représentent indépendamment un radical benzyle, acétyle, benzoyle, chlorobenzoyle, méthoxybenzoyle, nitrobenzoyle, allyle, chlorobenzyle, méthoxybenzyle ou nitrobenzyle ;
- ladite étape de couplage est réalisée en solution dans un solvant organique anhydre, à une température comprise entre -80°C et 40°C, pendant une durée de 1 minute à 8 heures, en présence d'un promoteur approprié choisi parmi :
   - une source d'ions halonium, associée ou non à un acide de Lewis ou un sel d'acide fort, dans le cas où X représente un groupement S(O)ₙR' tel que défini ci-dessus dans lequel n est égal à 0 ;
   - un acide de Lewis associé à une amine, dans le cas où X représente un groupement S(O)ₙR' tel que défini ci-dessus dans lequel n est égal à 1 ;
   - un acide de Brönstedt ou un acide de Lewis, dans le cas où X représente un groupement trichloroacétimidate.
- le produit de réaction ainsi obtenu, neutralisé et purifié étant soumis à un traitement de déprotection pour conduire, après purification, au Laminaribiose.

Il a été découvert, et ceci constitue le fondement de la présente invention, qu'il était possible de préparer par voie chimique, le Laminaribiose, avec un nombre limité d'étapes permettant d'obtenir un rendement global relativement élevé, par un choix judicieux du donneur et de l'accepteur de glycosyle, ainsi que du promoteur utilisé lors de la réaction de couplage.

Dans la description et les revendications, on entend :
- par radical alkyle ayant de 1 à 6 atomes de carbone, toute chaîne hydrocarbonée, linéaire ou ramifiée, comme par exemple un radical méthyle, éthyle, propyle, isopropyle, butyle, isobutyle, tertiobutyle, pentyle, isopentyle, hexyle, isohexyle ;
- par radical halogénoalkyle ayant de 1 à 6 atomes de carbone, tout radical alkyle dont 1 à 7 atomes d'hydrogène ont été substitués par 1 à 7 atomes d'halogène, comme par exemple un radical chlorométhyle, un radical bromométhyle, un radical trifluorométhyle, un radical trifluoro-2,2,2-éthyle, un radical pentafluoroéthyle, un radical heptafluoropropyle ;
- par radical aryle, un cycle aromatique ayant 5 ou 6 atomes de carbone ou hétéroatomes, comme par exemple un radical phényle, pyridyle, thiényle, furannyle, pyrimidyle.

Le donneur de glycosyle de formule (II) précitée ainsi que l'accepteur de glycosyle de formule (III) précitée peuvent être obtenus de façon relativement aisée. en une ou deux étapes, à partir du D-glucose.

Avantageusement, le donneur de glycosyle sera généralement choisi parmi les composés de formule (II) précitée dans laquelle :
- R₁: représente un radical choisi dans le groupe constitué des radicaux méthyle, chlorométhyle, trifluorométhyle, tertiobutyle, phényle, chlorophényle, méthoxyphényle et nitrophényle ;
- X: représente un radical choisi dans le groupe constitué des radicaux thiométhyle, thioéthyle, thiopropyle, thiophényle, thionitrophényle, thiopyridyle.

D'une façon générale, le promoteur utilisé au cours de l'étape de couplage précitée sera choisi parmi :
- le N-bromosuccinimide ou le N-iodosuccinimide, associé ou non à un acide de Lewis choisi parmi le chlorure ferrique, le ditriflate de cuivre, le ditriflate d'étain, l'éthérate de trifluorure de bore, le tétrachlorure d'étain ou de zirconium, le triflate de méthyle, le triflate de triméthyl- (ou de triéthyl-) silyle, le triflate d'argent, le ditriflate de cadmium, le ditriflate de cobalt, le ditriflate de nickel, le ditriflate de zinc, le tritriflate de bismuth, le tritriflate de fer, le tritriflate de gallium, ou à un sel d'acide fort tel que le triflate de tétrabutylammonium, dans le cas où X représente un groupement S(O)ₙR' tel que défini ci-dessus dans lequel n est égal à 0,
- un acide de Lewis choisi parmi l'anhydride triflique, le chlorure ferrique, le ditriflate de cuivre, le ditriflate d'étain, l'éthérate de trifluorure de bore, le tétrachlorure d'étain ou de zirconium, le triflate de méthyle, le triflate de triméthyl- (ou de triéthyl-) silyle, le triflate d'argent, le ditriflate de cadmium, le ditriflate de cobalt, le ditriflate de nickel, le ditriflate de zinc, le tritriflate de bismuth, le tritriflate de fer, le tritriflate de gallium, associé à une amine comme en particulier la di-tertiobutylméthylpyridine, dans le cas où X représente un groupement S(O)ₙR' tel que défini ci-dessus dans lequel n est égal à 1,
- un acide de Brönstedt comme en particulier l'acide triflique ou l'acide paratoluène sulfonique ou un acide de Lewis choisi parmi l'anhydride triflique, le chlorure ferrique, le ditriflate de cuivre, le ditriflate d'étain, l'éthérate de trifluorure de bore, le tétrachlorure d'étain ou de zirconium, le triflate de méthyle, le triflate de triméthyl- (ou de triéthyl-) silyle, le triflate d'argent, le ditriflate de cadmium, le ditriflate de cobalt, le ditriflate de nickel, le ditriflate de zinc, le tritriflate de bismuth, le tritriflate de fer, le tritriflate de gallium, dans le cas où X représente un groupement trichloroacétimidate.

Dans un mode de réalisation actuellement préféré du procédé selon l'invention :
- le donneur de glycosyle répond à la formule (II) précitée dans laquelle :
   - R₁: représente un radical phényle ; et
   - X: représente un radical S(O)ₙR' dans lequel n est égal à 0 et R' représente un radical éthyle ou phényle ;
- l'accepteur de glycosyle répond à la formule (III) précitée dans laquelle :
   R₂, R₃ et R₄, R₅ forment ensemble un radical cyclohexylidyle ou isopropylidyle.

Dans ce mode de réalisation particulier, le promoteur utilisé lors de la réaction de couplage est constitué d'un mélange de N-iodosuccinimide et de ditriflate d'étain, de préférence dans des proportions comprises entre 1:0,5 et 1:0,005.

D'une façon générale, l'étape de couplage précitée est réalisée en solution dans le dichlorométhane, le 1,2-dichloroéthane ou le toluène, de préférence en présence d'un tamis moléculaire, à une température comprise entre - 30°C et 30°C, pendant une durée de 1 minute à 6 heures, de préférence à 10°C pendant 30 minutes.

Les quantités respectives de donneur de glycosyle, d'accepteur de glycosyle et de promoteur pourront être facilement déterminées par l'homme de métier.

D'une façon générale, la réaction de couplage peut être mise en oeuvre en mettant en réaction :
- un équivalent d'accepteur de glycosyle
- un à deux équivalents de donneur de glycosyle ;
- un à deux équivalents de promoteur ;
- dans 5 à 200 équivalents en poids, par rapport à l'accepteur, d'un solvant.

Avantageusement, on utilisera un solvant organique comme le dichlorométhane, le 1,2-dichloroéthane ou le toluène, en présence d'un tamis moléculaire (destiné à piéger l'acide susceptible de se former au cours de la réaction) comme par exemple un tamis moléculaire de 4 Å, utilisé en une quantité de 10 à 200 mg/ml de solvant.

Le produit obtenu par la réaction de couplage précitée est généralement neutralisé, puis purifié.

La neutralisation peut être réalisée par addition d'une base organique, de préférence la triéthylamine, ou l'éthanolamine ou encore par addition d'une base inorganique de préférence l'hydrogénocarbonate ou le carbonate de sodium ou de potassium, suivie d'une filtration du sel obtenu.

La purification peut être réalisée :
soit par chromatographie, par exemple sur colonne de gel de silice ou de charbon actif,
soit par cristallisation fractionnée de préférence dans un solvant organique ou un mélange de solvants organiques comme l'éther éthylique, l'acétate d'éthyle, le cyclohexane ou l'éthanol.

Le produit de la réaction de couplage, neutralisé et purifié conduit, par un traitement de déprotection, suivi d'une purification, au Laminaribiose.

D'une façon générale, le traitement de déprotection précité comporte deux étapes, la première consistant en une déprotection partielle du produit de la réaction de couplage, par clivage des groupements acétals provenant de l'accepteur de glycosyle.

Dans le cadre du procédé conforme à la présente invention, le traitement de déprotection comprend :
a) le clivage des groupements acétals provenant de l'accepteur de glycosyle par un traitement acide en milieu aqueux ou hydroorganique, ou en présence d'une résine acide ;
b) la purification du produit ainsi obtenu ;
c) la transestérification ou l'hydrolyse du produit obtenu à l'étape b);
d) la purification du produit ainsi obtenu.

La réaction de clivage a) précitée sera réalisée de préférence en milieu hydro-organique acide, comme par exemple dans un mélange équivolumique d'acide trifluoroacétique et d'eau, à une température comprise entre 10 et 70°C pendant une durée de 1 heure à 10 jours et dans ce cas, le produit partiellement déprotégé obtenu sera purifié par cristallisation fractionnée de préférence dans le méthanol, ou par chromatographie.

L'acide acétique, l'acide oxalique, l'acide formique, l'acide sulfurique, l'acide chlorhydrique, l'acide phosphorique peuvent également être utilisés en lieu et place de l'acide trifluoroacétique.

Dans le cadre du procédé conforme à la présente invention l'étape de transestérification c) précitée sera réalisée dans un solvant alcoolique tel que le méthanol ou l'éthanol en présence d'une quantité catalytique de sodium ou de méthylate ou d'éthylate de sodium ou de potassium, pendant une durée de 1 minute à 10 jours.

Le produit de transestérification ainsi obtenu sera généralement purifié par un procédé comprenant :
d1) la neutralisation du produit obtenu à l'étape c)
d2) l'élimination de l'ester benzoïque formé, soit par évaporation azéotropique avec l'eau, soit par extraction avec un solvant organique
d3) la concentration sous pression réduite de la phase aqueuse résiduelle
d4) éventuellement, la lyophilisation ou la cristallisation dans un mélange hydroalcoolique du Laminaribiose ainsi obtenu.

D'autres caractéristiques et avantages de l'invention seront mieux compris à la lecture des exemples non-limitatifs suivants.

### Exemple 1 : Exemple de préparation d'un accepteur de glycosyle

Dans cet exemple, l'accepteur de glycosyle, à savoir le 1,2:5,6-di-*O*-cyclohexylidène-α-D-glucofuranose (composé de formule III dans laquelle R₂, R₃ et R₄, R₅ représentent un radical cyclohexylidyle) a été préparé en une seule étape à partir du D-glucose,

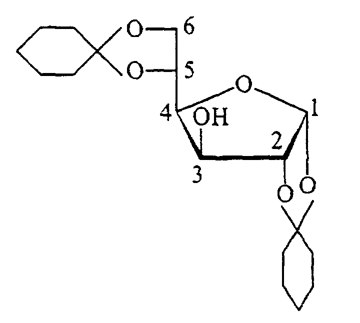

A 65 g de D-glucose (361 mmol ; 1 éq) et 85 ml (820 mmol ; 2,27 éq) de cyclohexanone dans 50 ml de 1,4-dioxane (587 mmol ; 1,62 éq) sont additionnés goutte à goutte 20 ml (375 mmol ; 1,03 éq) d'acide sulfurique concentré à température ambiante. L'addition terminée, au bout de 30 minutes, le produit précipite dans le milieu réactionnel. Le précipité est brisé, filtré et lavé à l'eau. Le produit brut est alors purifié par recristallisation dans le cyclohexane pour conduire à 104 grammes de 1,2 : 5,6-di-*O*-cyclohexylidène-α-D-glucofuranose.

Rendement (%) : 85

Solide blanc.

F(°C) = 134-136

CCM : R_{f} = 0.8 (dichlorométhane / méthanol (9/1 ; v/v)).

RMN ¹³C (CDCl₃, 101 MHz) δ (ppm) : 112,49, 110,32 (C quat.) ; 104,93 (C1); 84,62 (C2) ; 81,23 (C4) ; 75,39 (C3) ; 73,27 (C5) ; 67,40 (C6) ; 36,50, 36,48, 35,69, 34,65, 25,09, 24,94, 24,08, 23,95, 23,82, 23,63, (CH₂).

RMN ¹H (CDCl₃, 400 MHz) δ (ppm) : 5,93, (d, 1H, H1, J_{H1-H2} = 3,6 Hz) ; 4,50 (d, 1H, H2, J_{H2-H1} = 3,6 Hz) ; 4,34-4,30 (m, 2H, H3, H5) ; 4,14 (dd, 1H, H6, J_{H6-H5} = 6,2 Hz, J_{H6-H6'} = 8,6 Hz) ; 4,04 (dd, 1H, H4, J_{H4-H3} = 2,8 Hz , J_{H4-H5} = 7,6 Hz) ; 3,95 (dd, 1H, H6', J_{H6'-H5} = 5,4 Hz, J_{H6'-H6} = 8,6 Hz) ; 1,70-1,36 (m, 20H, CH₂).

### Exemple 2 : Exemple de préparation d'un donneur de glycosyle :

Dans cet exemple, le donneur de glycosyle (composé de formule II dans laquelle R₁ est un phényle et X un groupe S(O)ₙR' dans lequel n = 0 et R' représente un groupement éthyle) a été préparé en deux étapes.

### a) Préparation du 1,2,3,4,6-penta-O-benzoyl-D-glucopyranose

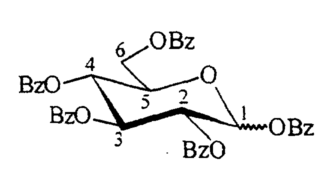

100 g de D-glucose (555 mmol ; 1 éq) sont dissous dans 21 de pyridine et le milieu réactionnel est chauffé à reflux pendant 1 heure puis additionné à chaud (60-70°C) de 387 ml (3330 mmol ; 6 éq) de chlorure de benzoyle. Après addition, le milieu est dilué à l'eau, le produit précipite et est filtré et rincé à l'eau jusqu'à neutralité. Après séchage, il est purifié par recristallisation dans l'acétate d'éthyle.

Rendement (%) : 100

Solide blanc.

F (°C) = 164-166 (anomère β) ; 188-191 (anomère α).

CCM : R_{f}= 0,3 (éther de pétrole/acétate d'éthyle (8/2 ; v/v)).

RMN ¹³C (CDCl₃, 101 MHz) δ (ppm) : anomère β : 166,17, 165,74, 165,19, 165,16, 164,66 (C=O) ; 92,74 (C1) ; 73,21, 72,85 (C3, C5) ; 70,87 (C2) ; 69,08 (C4); 62,71 (C6); anomère α: 166,15, 165,96, 165,42, 165,18, 164,47 (C=O) ; 90,08 (C1) ; 70,54, 70,51, 70,45 (C2, C3, C5) ; 68,83 (C4) ; 62,49 (C6).

RMN ¹H (CDCl₃, 400 MHz) δ (ppm) : 8,20-7,16 (m, 25H, H arom.) ; anomère β : 6,30 (d, 1H, H1, J_{H1-H2} = 8,0 Hz) ; 6,05 (t, 1H, H3, J_{H3-H2} = J_{H3-H4} = 9,5 Hz) ; 5,87 (dd, 1H, H2, J_{H2-H1} = 8,0 Hz, J_{H2-H3} = 9,5 Hz) ; 5,84 (t, 1H, H4, J_{H4-H3} = J_{H4-H5} = 9,6 Hz) ; 4,66 (dd, 1H, H6, J_{H6-H5} = 2,9 Hz, J_{H6-H6'} = 12,3 Hz) ; 4,52 (dd, 1H, H6', J_{H6'-H5} = 4,7 Hz, J_{H6'-H6} = 12,3 Hz) ; 4,41 (ddd, 1H, H5, J_{H5-H4} = 9,7 Hz, J_{H5-H6} = 3,0 Hz, J_{H5-H6'} = 4,6 Hz) ; anomère α : 6,85 (d, 1H, H1, J_{H1-H2} = 3,8 Hz) ; 6,33 (t, 1H, H3, J_{H3-H2} = J_{H3-H4} = 10,0 Hz) ; 5,88 (t, 1H, H4, J_{H4-H3} = J_{H4-H5} = 9.8 Hz) ; 5,69 (dd, 1H, H2, J_{H2-H1} = 3,8 Hz, J_{H2-H3} = 10,3 Hz) ; 4,65-4,60 (m, 2H, H5, H6) ; 4,48 (dd, 1H, H6', J_{H6'-H5} = 5,0 Hz, J_{H6'-H6} = 13,0 Hz).

### b) Préparation du 2,3,4,6-tétra-O-benzoyl-1-thio-D-glucopyranoside d'éthyle

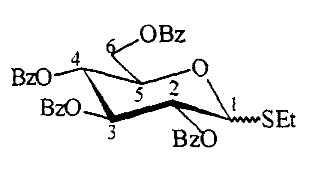

100 g de glucose perbenzoylé (143 mmol ; 1 éq) sont dissous dans 2 l de dichlorométhane et le milieu réactionnel est porté au reflux puis additionné de 12,7 ml d'éthanethiol (171 mmol ; 1,2 éq) et de 54,2 ml (429 mmol ; 3 éq) d'éthérate de trifluorure de bore. Après 2 heures de réaction au total, le milieu est dilué avec du dichlorométhane, lavé avec une solution d'hydrogénocarbonate de sodium à 5% puis à l'eau jusqu'à pH neutre. Le produit est recristallisé dans l'acétate d'éthyle et on recueille ainsi 73 grammes de produit.

Rendement (%) : 80

Solide blanc.

F (°C) = 133-134 (anomère α)

CCM : R_{f} = 0,4 (anomère β); 0,5 (anomère α) (éther de pétrole/acétate d'éthyle (8/2 ; v/v)).

RMN ¹³C (CDCl₃, 101 MHz) δ (ppm) : 166,23, 166,18, 165,87, 165,71, 165,50, 165,38, 165,27 (C=O) ; anomère α : 82,10 (C1) ; 71,74 (C2) ; 70,99 (C3) ; 69,62 (C4) ; 68,21 (C5) ; 63,14 (C6) ; 24,34 (CH₂) ; 14,71 (CH₃) ; anomère β : 84,02 (C1) ; 76,37 (C5) ; 74,19 (C3) ; 70,68 (C2) ; 69,71 (C4) ; 63,42 (C6) ; 24,47 (CH₂) ; 15,01 (CH₃).

RMN ¹H (CDCl₃, 400 MHz) δ (ppm) : 8,06-7,26 (2m, 40H, H arom.) ; anomère α : 6,08 (t, 1H, H3, J_{H3-H2} = J_{H3-H4} = 9,9 Hz) ; 5,95 (d, 1H, H1, J_{H1-H2} = 5,8 Hz) ; 5,68 (t, 1H, H4, J_{H4-H3} = J_{H4-H5} = 9,9 Hz) ; 5,51 (dd, 1H, H2, J_{H2-H1} = 5,8 Hz, J_{H2-H3} = 10,2 Hz) ; 4,88 (ddd, 1H, H5, J_{H5-H4} = 10,2 Hz, J_{H5-H6} = 2,8 Hz, J_{H5-H6'} = 5,4 Hz) ; 4,60 (dd, 1H, H6, J_{H6-H5} = 2,8 Hz, J_{H6-H6'} = 12,2 Hz) ; 4,52 (dd, 1H, H6', J_{H6'-H5} = 5,4 Hz, J_{H6'-H6} = 12,2 Hz) ; 2,62 (qd, 2H, CH₂, J = 7,4 Hz, J = 9,6 Hz) ; 1,25 (t, 3H, CH₃, J = 7,4 Hz) ; anomère β : 5,93 (t, 1H, H3, J_{H3-H2} = J_{H3-H4} = 9,7 Hz) ; 5,68 (t, 1H, H4, J_{H4-H3} = J_{H4-H5} = 9,8 Hz) ; 5,57 (t, 1H, H2, J_{H2-H1} = J_{H2-H3} = 9,7 Hz) ; 4,87 (d, 1H, H1, J_{H1-H2} = 10 Hz) ; 4,64 (dd, 1H, H6, J_{H6-H5} = 3,1 Hz, J_{H6-H6'} = 12,1 Hz) ; 4,50 (dd, 1H, H6', J_{H6'-H5} = 5,5 Hz, J_{H6'-H6} = 12,1 Hz) ; 4,18 (ddd 1H, H5, J_{H5-H4} = 10,0 Hz, J_{H5-H6} = 3,0 Hz, J_{H5-H6'} = 5,5 Hz) ; 2,77 (qd, 2H, CH₂, J = 7,4 Hz, J = 9,6 Hz) ; 1,26 (t, 3H, CH₃, J = 7,4 Hz).

### Exemple 3 : Exemple d'une réaction de couplage selon l'invention

On a réalisé une réaction de couplage entre le donneur de l'exemple 2 et l'accepteur de l'exemple 1 pour obtenir le composé nouveau suivant :

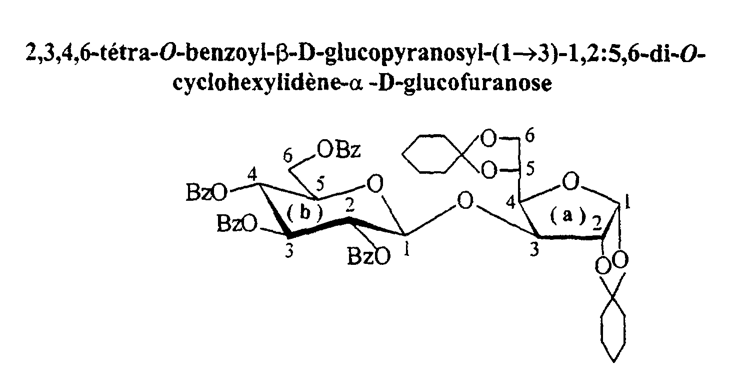

64 g (100 mmol; 1,06 éq) de 2,3,4,6-tétra-*O* benzoyl-1-thio-D-glucopyranoside d'éthyle, 32 g (94 mmol ; 1 éq) de 1,2:5,6-di-*O*-cyclohexylidène-α-D-glucofuranose, 22,5 g (100 mmol ; 1.06 éq) de *N*-iodosuccinimide et 400 g de tamis moléculaire 4Å sont introduits dans un ballon à l'abri de la lumière puis dissous dans 400 ml de dichlorométhane anhydre à une température de 10°C, et 3,92 grammes (9,4 mmol ; 0,1 éq) de ditriflate d'étain sont alors ajoutés. Après 1 heure de réaction, le milieu est neutralisé par de la triéthylamine, filtré, concentré et la purification sur colonne de gel de silice (flash, éluant : toluène/acétate d'éthyle (95/5 puis 9/1 : v/v)) permet de recueillir 60,5 grammes de produit.

Rendement (%) : 70

Solide blanc.

CCM : R_{f}= 0,4 (toluène/acétate d'éthyle (9/1 ; v/v)).

RMN ¹³C (CDCl₃, 101 MHz) δ (ppm) : 166,19, 165,86, 165,20, 164,83 (C =O) ; 112,71, 109,32 (C quat.); 104,66 (C1a) ; 100,08 (C1b) ; 82,83 (C2a) ; 81,68 (C3a); 80,66 (C4a); 72,79 (C3b) ; 72,63 (C5b); 72,46 (C5a) ; 71,99 (C2b); 69,57 (C4b); 66,26 (C6a); 63,12 (C6b); 36,43, 36,33, 35,49, 34,61, 25,23, 24,84, 24,17, 23,89, 23,86, 23,54 (CH₂).

RMN ¹H (CDCl₃, 400 MHz) δ (ppm) : 8,03-7,26 (2m, 20H, H arom.); 5,90 (t, 1H, H3b, J_{H3b-H2b} = J_{H3b-H4b} = 9,6 Hz); 5,71 (t, 1H, H4b, J_{H4b-H3b} = J_{H4b-H5b} = 9,6 Hz); 5,52 (dd, 1H, H2b, J_{H2b-H1b} = 8,2 Hz, J_{H2b-H3b} = 9,4 Hz) ; 5,45 (d, 1H, H1a, J_{H1a-H2a} = 3,6 Hz); 4,99 (d, 1H, H1b, J_{H1b-H2b} = 7,8 Hz) ; 4,64 (dd, 1H, H6b, J_{H6b-H5b} = 2,4 Hz, J_{H6b-H6'b} = 12,2 Hz); 4,49 (dd, 1H, H6'b, J_{H6'b-H5b} = 5,0 Hz, J_{H6'b-H6b} = 12,2 Hz) ; 4,38 (q, 1H, H5a, J_{H5a-H4a} = J_{H5a-H6a} = J_{H5a-H6'a} = 6,1 Hz) ; 4,35 (d, 1H, H3a, J_{H3a-H4a} = 3,0 Hz) ; 4,32 (d, 1H, H2a, J_{H2a-H1a} = 3,6 Hz) ; 4,18 (dd, 1H, H4a, J_{H4a-H3a} = 2,9 Hz, J_{H4a-H5a} = 6,3 Hz) ; 4,11 (ddd, 1H, H5b, J_{H5b-H4b} = Hz, J_{H5b-H6b} = Hz, J_{H5b-H6'b} = Hz) ; 4,03 (dd, 1H, H6a, J_{H6a-H5a} = 6,8 Hz, J_{H6a-H6'a} = 8,3 Hz) ; 3,94 (dd, 1H, H6'a, J_{H6'a-H5a} = 5,6 Hz, J_{H6'a-H6a} = 8,3 Hz) ; 1,62-1,30 (m, 20H, CH₂).

### Exemple 4 : Autre exemple d'une réaction de couplage selon l'invention

Dans cet exemple, on a préparé par une réaction de couplage le :

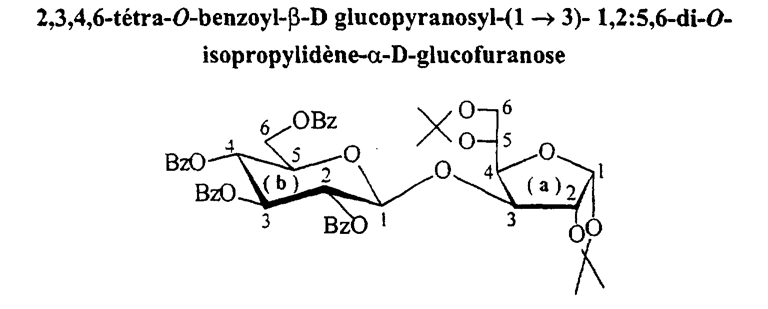

Dans cet exemple, le composé accepteur de glycosyle est le diacétone D-glucose qui peut être facilement obtenu à partir du D-glucose et de l'acétone.

64 g (100 mmol; 1,06 éq) de 2,3,4,6-tétra-*O*-benzoyl-1-thio-D-glucopyranoside d'éthyle préparé à l'exemple 2, 24,5 g (94 mmol ; 1 éq) de diacétone-D-glucose, 22,5 g (100 mmol ; 1,06 éq) de *N*-iodosuccinimide et 400 g de tamis moléculaire 4Å sont introduits dans un ballon à l'abri de la lumière puis dissous dans 400 ml de dichlorométhane anhydre. Le milieu réactionnel est refroidi à 0°C et additionné de 3,92 g (9,4 mmol ; 0,1 éq) de ditriflate d'étain. Après 20 minutes de réaction, le milieu est neutralisé par de la triéthylamine, filtré, concentré et la purification sur colonne de gel de silice (flash, éluant : dichlorométhane/acétate d'éthyle (95/5: v/v)) permet de recueillir 28 grammes de produit.

Rendement (%) : 35

Solide blanc.

F (°C) = 90-94

CCM : R_{f}= 0,5 (toluène/acétate d'éthyle (8/2 ; v/v)).

RMN ¹³C (CDCl₃, 101 MHz) δ (ppm) : 166,16, 165,85, 165,19, 164,81 (C =O) ; 112,00, 108,72 (C quat.) ; 104,99 (C1a) ; 99,99 (C1b) ; 82,78 (C2a) ; 81,48 (C3a) ; 80,47 (C4a) ; 73,05 (C5a) ; 72,72 (C3b) ; 72,65 (C5b) ; 71,85 (C2b) ; 69,54 (C4b) ; 66,25 (C6a) ; 62,98 (C6b) ; 26,73, 26,67, 26,03, 25,15 (CH₃).

RMN ¹H (CDCl₃, 400 MHz) δ (ppm) : 8,03-7,26 (2m, 20H, H arom.) ; 5,90 (t, 1H, H3b, J_{H3b-H2b} = J_{H3b-H4b} = 9,7 Hz) ; 5,71 (t, 1H, H4b, J_{H4b-H3b} = J_{H4b-H5b} = 9,7 Hz) ; 5,50 (dd, 1H, H2b, J_{H2b-H1b} = 7,8 Hz, J_{H2b-H3b} = 9,7 Hz) ; 5,48 (d, 1H, H1a, J_{H1a-H2a} = 3,7 Hz) ; 4,98 (d, 1H, H1b, J_{H1b-H2b} = 7,8 Hz) ; 4,66 (dd, 1H, H6b, J_{H6b-H5b} = 3,2 Hz, J_{H6b-H6'b} = 12,2 Hz) ; 4,50 (dd, 1H, H6'b, J_{H6'b-H5b} = 5,1 Hz, J_{H6'b-H6b} = 12,2 Hz) ; 4,38 (m, 1H, H5a) ; 4,34 (d, 1H, H2a, J_{H2a-H1a} = 3,7 Hz) ; 4,33 (d, 1H, H3a, J_{H3a-H4a} = 3,2 Hz) ; 4,23 (dd, 1H, H4a, J_{H4a-H3a} = 3,2 Hz, J_{H4a-H5a} = 5,5 Hz) ; 4,16 (ddd, 1H, H5b, J_{H5b-H4b} = 9,7 Hz, J_{H5b-H6b} = 3,2 Hz, J_{H5b-H6'b} = 5,1 Hz) ; 4.04 (dd, 1H, H6a, J_{H6a-H5a} = 6,5 Hz, J_{H6a-H6'a} = 8,6 Hz) ; 3,97 (dd, 1H, H6'a, J_{H6'a-H5a} = 5,7 Hz, J_{H6'a-H6a} = 8,6 Hz) ; 1,42, 1,37, 1,25, 1,12 (4s, 12H, CH₃).

### Exemple 5 : Cet exemple illustre la réaction de déprotection conduisant au Laminaribiose réalisée en deux étapes selon l'invention

### a) 2,3,4,6-tétra-O-benzoyl-β-D-glucopyranosyl-(1 → 3)-D-glucopyranose

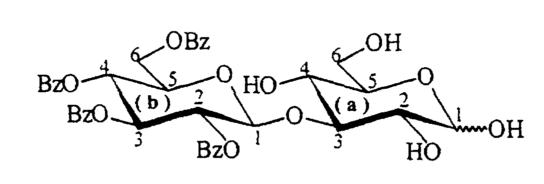

82 g de 2,3,4,6-tétra-*O*-benzoyl-β-D-glucopyranosyl-(1 → 3)-1,2:5,6-di-*O*-cyclohexylidène-α -D-glucofuranose préparé à l'exemple 3 sont dissous dans 600 ml d'un mélange équivolumique d'acide trifluoroacétique et d'eau auquel a été ajouté 60 ml de tétrahydrofurane pour obtenir une bonne dissolution du produit dans le milieu réactionnel. Après 1 jour de réaction à 40°C, le produit est précipité par dilution du milieu réactionnel avec de l'eau, filtré, rincé à l'eau jusqu'à neutralité, séché et purifié par cristallisation dans le méthanol. On obtient ainsi 54 g du produit attendu.

Rendement (%) : 80

Solide blanc.

F(°C)=183-186 ; 197-200 (2 anomères α et β).

CCM : R_{f}= 0,6 (dichlorométhane/méthanol (9/1 ; v/v)).

RMN ¹³C (pyr. d⁵, 101 MHz) δ(ppm): 165,90 ,165,87, 165,81, 165,65, 165,58, 165,39, 165,37 (C=O) ; 133,41, 133,22, 133,00, 132,96, 132,93 (C ipso arom.) ; 130,05-128,28 (C arom.) ; 101,89, 101,70 (C1b); 98,46 (C1aβ); 93,59 (C1aα); 86,48 (C3aβ); 84,69 (C3aα) ; 77,78 (C5aβ) ; 75,80 (C2aβ) ; 74,09, 74.06 (C3b) ; 73,24 (C2aα) ; 73,04, 72,95, 72,90 (C5aα, C2b) ; 72.04 (C5b) ; 70,36, 70,29 (C4b) ; 69,55, 65,45 (C4a) ; 63,34, 63,24 (C6b) ; 62,51, 62,36 (C6a).

RMN ¹H (pyr. d⁵, 400 MHz) δ(ppm): 8,29-7,09 (3m, 40H. H arom.); 6,58 (t, 1H, H3b, J_{H3b-H4b} = J_{H3b-H2b} = 9,5 Hz) ; 6,52 (t, 1H, H3b, J_{H3b-H4b} = J_{H3b-H2b} = 9,5 Hz) ; 6,32 (d, 1H, H1b, J_{H1b-H2b} = 8,0 Hz); 6,23-6,10 (m, 5H, 2 H4b, H1b, 2 H2b); 5,69 (d, 1H, H1aα, J_{H1aα-H2aα} = 3,4 Hz); 5,22 (d, 1H, H1aβ, J_{H1aβ-H2aβ} = 7,4 Hz); 4,94 (dd, 1H, H6b, J_{H6b-H5b} = 2,7 Hz, J_{H6b-H6'b} = 12,1 Hz); 4,88-4,69 (m, 6H, H6b, H3aα, H6b, H5aα, H6b, H5b); 4,52-4,57 (m, 3H, H6aα, H6aβ, H5b); 4,45 (t, 1H, H3aβ, J_{H3aβ-H4aβ} = J_{H3aβ-H2aβ} = 9,1 Hz); 4,34-4,26 (m, 2H, H_{6'aα}, H6'aβ); 4,20-4,13 (m, 2H, H4aα, H4aβ); 4,10-4,03 (m, 2H, H2aα, H2aβ); 3,97-3,93 (ddd, 1H, H5aβ).

### b) Obtention du Laminaribiose

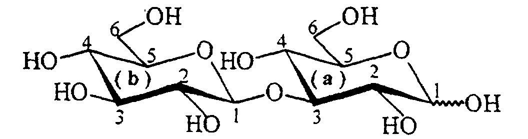

40 g (52,7 mmol ; 1 éq) de 2,3,4,6-tetra-*O*-benzoyl-β-D-glucopyranosyl-(1 → 3)-D-glucopyranose sont traités par 1 l d'une solution de méthylate de sodium (0,1 éq de sodium) préalablement préparée en dissolvant 6 g de sodium dans 40 l de méthanol anhydre. Après 4 jours de réaction à 35°C, le milieu est dilué avec de l'eau puis neutralisé avec de la résine acide IR 120, filtré et concentré. Après lyophilisation, 18 g de Laminaribiose sont recueillis. Le produit peut être cristallisé dans un mélange méthanol-eau ou éthanol-eau.

Rendement (%) : 100

Solide blanc.

F (°C) = 199-204 (éthanol-eau ; Bächli, Percival, *J. Chem*. *Soc.,* 1952, 1243 : 196-206 ; Takeo, *Carbohydr*. *Res.,* 1979, 77, 245 : 202-204).

F (°C) = 124-130 (Laminaribiose lyophilisé).

[α]_{D}²⁰ = + 19,3° (20 min) → + 18,7° (24 h ; c = 1,0 ; eau) (Takeo, *Carbohydr. Res*., 1979, 77, 245 : [α]_{D}²⁰ = + 15,5° (10 min) → + 18,6° (24 h ; c = 2,3 ; eau)).

CCM : R_{f} = 0,1 (acétate d'éthyle/isopropanol/méthanol/eau (10/6/1/1 ; v/v).

RMN ¹³C (DMSO d⁶, 101 MHz) δ (ppm) : anomère β : 104,16 (C1b); 96,33 (C1a); 88,43 (C3a) ; anomère α: 104,04 (C1b); 91,78 (C1a); 85,19 (C3a) ; 76,93, 76,87, 76,24, 76,10, 76,07, 73,87, 73,84, 73,50, 71,90, 70,90, 70,18, 70,13, 68,66, 68,57, 61,10, 60,92.

RMN ¹H (D₂O, 400 MHz) δ (ppm) : 5,11 (d, 1H, H1aα, J_{H1aα-R2aα} = 3,8 Hz) ; 4,61 (d, 1H, H1b, J_{H1b-H2b} = 7,9 Hz) ; 4,60 (d, 1H, H1b, J_{H1b-H2b} = 7,6 Hz) ; 4,55 (d, 1H, H1aβ, J_{H1aβ-H2aβ} = 8,0 Hz) ; 3,81-3,57 (m, 12H) ; 3,42-3,22 (m, 12H).

### Exemple 6 : Caractérisation du Laminaribiose

Afin de caractériser le produit obtenu, le Laminaribiose synthétisé a été acétylé en présence d'anhydride acétique (12 éq) dans la pyridine puis recristallisé dans l'éthanol. On a obtenu exclusivement l'anomère β, c'est-à-dire le :

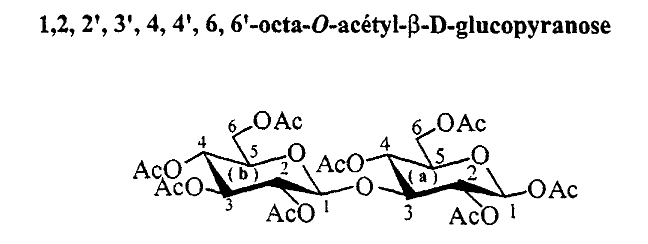

Rendement (%) : 100

Solide blanc.

F (°C) = 165-167 (Takeo, *Carbohydr*. *Res*., 1979, 77, 245: 161-162).

[α]_{D}²⁰ = - 27° (c = 1,0 ; chloroforme) (Takeo, *Carbohydr*. *Res.,* 1979, 77, 245 : [α]_{D}²⁰ = - 27,6° (c = 1,2 ; chloroforme)).

CCM : R_{f}= 0,3 (toluène/acétate d'éthyle (1/1 ; v/v)).

RMN ¹³C (CDCl₃, 101 MHz) δ (ppm) : 170,79, 170,57, 170,45, 169,38, 169,37, 169,29, 169,20, 168,92 (C=O) ; 101,02 (C1b); 91,80 (C1a) ; 78,92 (C3a) ; 72,98, 72,87 (C5a, C3b ou vice versa) ; 71,15 (C2b) ; 67,99 (C4b) ; 67,56 (C4a) ; 61,73, 61,71 (C6a, C6b ou vice versa) ; 20,91, 20,88, 20,84, 20,74, 20,64, 20,62,20,52, 20,39 (CH₃).

RMN ¹H (CDCl₃, 400 MHz) δ (ppm) : 5,61 (d, 1H, H1a, J_{H1a-H2a} = 8,4 Hz) ; 5,13 (t, 1H, H3b, J_{H3b-H2b} = J_{H3b-H4b} = 9,4 Hz) ; 5,12 (dd, 1H, H2a, J_{H2a-H1a} = 8,4 Hz, J_{H2a-H3a} = 9,5 Hz) ; 5,06 (t, 1H, H4b, J_{H4b-H3b} = J_{H4b-H5b} = 9,6 Hz) ; 5,01 (t, 1H, H4a, J_{H4a-H3a} = J_{H4a-H5a} = 9,6 Hz) ; 4,90 (dd, 1H, H2b, J_{H2b-H1b} = 8,1 Hz, J_{H2b-H3b} = 9,3 Hz) ; 4,59 (d, 1H, H1b, J_{H1b-H2b} = 8,1 Hz) ; 4,37 (dd, 1H, H6a, J_{H6a-H5a} = 4,3 Hz, J_{H6a-H6'a} = 12,4 Hz) ; 4,21 (dd, 1H, H6b, J_{H6b-H5b} = 4,6 Hz, J_{H6b-H6'b} = 12,4 Hz) ; 4,13 (dd, 1H, H6'b, J_{H6'b-H5b} = 2,2 Hz, J_{H6'b-H6b} = 12,4 Hz) ; 4,05 (dd, 1H, H6'a, J_{H6'a-H5a} = 2,2 Hz, J_{H6'a-H6a} = 12,4 Hz) ; 3,93 (t, 1H, H3a, J_{H3a-H2a} = J_{H3a-H4a} = 9,4 Hz) ; 3,78 (ddd, 1H, H5a, J_{H5a-H4a} = 10,1 Hz, J_{H5a-H6a} = 2,2 Hz, J_{H5a-H6'a} = 4,6 Hz) ; 3,68 (ddd, 1H, H5b, J_{H5b-H4b} = 9,9 Hz, J_{H5b-H6b} = 4,3 Hz, J_{H5b-H6'b} = 2,3 Hz) ; 2,12, 2,09, 2,08, 2,03, 2,00, 1,98 (6s, 24H, CH₃).

## Revendications

1. Procédé de préparation du Laminaribiose comprenant une étape de couplage glycosidique entre un donneur et un accepteur de glycosyle, **caractérisé en ce que** :
• le donneur de glycosyle se présente sous forme pyranosique et répond à la formule (II) 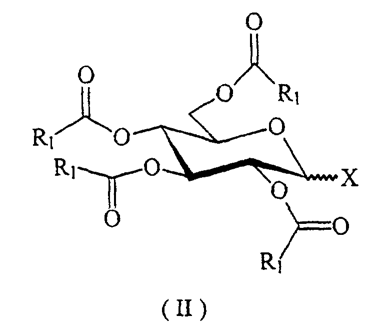 dans laquelle
R₁ représente :
- un radical alkyle ou halogénoalkyle ayant de 1 à 6 atomes de carbone ;
- un radical aryle non substitué ou substitué par un ou plusieurs groupements choisis parmi un atome d'halogène, un radical alcoxy ayant de 1 à 6 atomes de carbone ou un groupement nitro ;
X représente un groupe partant nucléofuge choisi parmi :
- un groupement de formule S(O)ₙR' dans laquelle R' représente un radical alkyle ayant de 1 à 6 atomes de carbone, un radical aryle non substitué ou substitué par un groupement alcoxy ayant de 1 à 6 atomes de carbone, un groupement nitro ou acétamide, et n est un nombre entier égal à 0 ou 1 ;
- un groupement trichloroacétimidate
• l'accepteur de glycosyle se présente sous forme furanosique et répond à la formule (III) 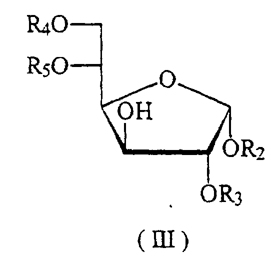 dans laquelle :
R₂ et R₃ forment ensemble un radical méthylidyle, éthylidyle, trichloroéthylidyle, isopropylidyle, hexafluoroisopropylidyle, cyclopentylidyle, cyclohexylidyle, cycloheptylidyle, butylidyle, 1-tertiobutyléthylidyle, 1-phényléthylidyle, benzylidyle, méthoxybenzylidyle, 1-phénylbenzylidyle ;
R₄ et R₅ forment ensemble un radical méthylidyle, éthylidyle, trichloroéthylidyle, isopropylidyle, hexafluoroisopropylidyle, cyclopentylidyle, cyclohexylidyle, cycloheptylidyle, butylidyle, 1-tertiobutyléthylidyle, 1-phényléthylidyle, benzylidyle, méthoxybenzylidyle, 1-phénylbenzylidyle ou représentent indépendamment un radical benzyle, acétyle, benzoyle, chlorobenzoyle, méthoxybenzoyle, nitrobenzoyle, allyle, chlorobenzyle, méthoxybenzyle ou nitrobenzyle ;
• ladite étape de couplage est réalisée en solution dans un solvant organique anhydre, à une température comprise entre -80°C et 40°C, pendant une durée de 1 minute à 8 heures, en présence d'un promoteur approprié choisi parmi :
- une source d'ions halonium, associée ou non à un acide de Lewis ou un sel d'acide fort, dans le cas où X représente un groupement S(O)ₙR' tel que défini ci-dessus dans lequel n est égal à 0 ;
- un acide de Lewis associé à une amine, dans le cas où X représente un groupement S(O)ₙR' tel que défini ci-dessus dans lequel n est égal à 1 ;
- un acide de Brönstedt ou un acide de Lewis, dans le cas où X représente un groupement trichloroacétimidate.
• le produit de réaction ainsi obtenu, neutralisé et purifié étant soumis à un traitement de déprotection pour conduire, après purification, au Laminaribiose.

2. Procédé selon la revendication 1, **caractérisé en ce que** :
• le donneur de glycosyle répond à la formule (II) précitée dans laquelle :
R₁ représente un radical choisi dans le groupe constitué des radicaux méthyle, chlorométhyle, trifluorométhyle, tertiobutyle, phényle, chlorophényle, méthoxyphényle et nitrophényle ;
X représente un radical choisi dans le groupe constitué des radicaux thiométhyle, thioéthyle, thiopropyle, thiophényle, thionitrophényle, thiopyridyle.

3. Procédé selon la revendication 1 ou 2, **caractérisé en ce que** le promoteur précité est choisi parmi :
- le N-bromosuccinimide ou le N-iodosuccinimide, associé ou non à un acide de Lewis choisi parmi le chlorure ferrique, le ditriflate de cuivre, le ditriflate d'étain, l'éthérate de trifluorure de bore, le tétrachlorure d'étain ou de zirconium, le triflate de méthyle, le triflate de triméthyl- (ou de triéthyl-) silyle, le triflate d'argent, le ditriflate de cadmium, le ditriflate de cobalt, le ditriflate de nickel, le ditriflate de zinc, le tritriflate de bismuth, le tritriflate de fer, le tritriflate de gallium, ou à un sel d'acide fort tel que le triflate de tétrabutylammonium, dans le cas où X représente un groupement S(O)ₙR' tel que défini ci-dessus dans lequel n est égal à O,
- un acide de Lewis choisi parmi l'anhydride triflique, le chlorure ferrique, le ditriflate de cuivre, le ditriflate d'étain, l'éthérate de trifluorure de bore, le tétrachlorure d'étain ou de zirconium, le triflate de méthyle, le triflate de triméthyl- (ou de triéthyl-) silyle, le triflate d'argent, le ditriflate de cadmium, le ditriflate de cobalt, le ditriflate de nickel, le ditriflate de zinc, le tritriflate de bismuth, le tritriflate de fer, le tritriflate de gallium, associé à une amine comme en particulier la di-tertiobutylméthylpyridine, dans le cas où X représente un groupement S(O)ₙR' tel que défini ci-dessus dans lequel n est égal à 1,
- un acide de Brönstedt comme en particulier l'acide triflique ou l'acide paratoluène sulfonique ou un acide de Lewis choisi parmi l'anhydride triflique, le chlorure ferrique. le ditriflate de cuivre, le ditriflate d'étain, l'éthérate de trifluorure de bore, le tétrachlorure d'étain ou de zirconium, le triflate de méthyle, le triflate de triméthyl- (ou de triéthyl-) silyle, le triflate d'argent, le ditriflate de cadmium, le ditriflate de cobalt, le ditriflate de nickel, le ditriflate de zinc, le tritriflate de bismuth, le tritriflate de fer, le tritriflate de gallium, dans le cas où X représente un groupement trichloroacétimidate.

4. Procédé selon la revendication 2 ou 3, **caractérisé en ce que** :
• le donneur de glycosyle répond à la formule (II) précitée dans laquelle :
R₁ représente un radical phényle ; et
X représente un radical S(O)ₙR' dans lequel n est égal à O et R' représente un radical éthyle ou phényle ;
• l'accepteur de glycosyle répond à la formule (III) précitée dans laquelle :
R₂, R₃ et R₄, R₅ forment ensemble un radical cyclohexylidyle ou isopropylidyle.

5. Procédé selon la revendication 4, **caractérisé en ce que** :
• le promoteur précité est un mélange de N-iodosuccinimide et de ditriflate d'étain de préférence dans des proportions comprises entre 1:0,5 et 1:0,005.

6. Procédé selon l'une des revendications 1 à 5, **caractérisé en ce que** le traitement de déprotection précité comprend :
a) le clivage des groupements acétals provenant de l'accepteur du glycosyle par un traitement acide en milieu aqueux ou hydroorganique, ou en présence d'une résine acide ;
b) la purification du produit ainsi obtenu ;
c) la transestérification ou l'hydrolyse du produit obtenu à l'étape b) ;
d) la purification du produit ainsi obtenu.

7. Procédé selon la revendication 6, **caractérisé en ce que** l'étape a) précitée est réalisée par la mise en réaction du produit de couplage neutralisé et purifié dans un mélange, de préférence équivolumique, d'acide trifluoroacétique et d'eau à une température comprise entre 10 et 70 °C pendant une durée 1 heure à 10 jours et **en ce que** l'étape de purification b) précitée est réalisée par cristallisation fractionnée, de préférence dans le méthanol.

8. Procédé selon la revendication 6 ou 7, **caractérisé en ce que** l'étape de transestérification précitée est réalisée dans un solvant alcoolique tel que le méthanol ou l'éthanol, en présence d'une quantité catalytique de sodium ou de méthylate ou d'éthylate de sodium ou de potassium pendant une durée de 1 minute à 10 jours et **en ce que** l'étape de purification d) précitée comprend :
d1) la neutralisation du produit obtenu à l'étape c)
d2) l'élimination de l'ester benzoïque formé, soit par évaporation azéotropique avec l'eau, soit par extraction avec un solvant organique
d3) la concentration sous pression réduite de la phase aqueuse résiduelle
d4) éventuellement, la lyophilisation ou la cristallisation dans un mélange hydroalcoolique du Laminaribiose ainsi obtenu.

9. Procédé selon l'une des revendications précédentes, **caractérisé en ce que** l'étape de couplage précitée est réalisée en solution dans le dichlorométhane, le 1,2-dichloroéthane ou le toluène, de préférence en présence d'un tamis moléculaire, à une température comprise entre - 30°C et 30°C, pendant une durée de 1 minute à 6 heures, de préférence à 10°C pendant 30 minutes.

10. Intermédiaires de synthèse du Laminaribiose **caractérisés en ce qu'**ils sont choisis parmi les composés suivants :

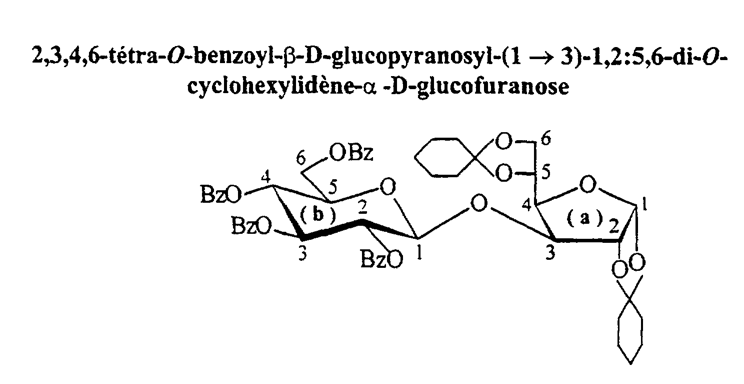

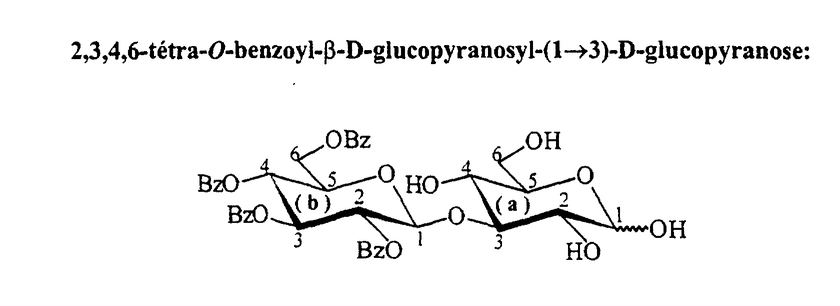

## Patentansprüche

1. Verfahren zur Herstellung von Laminaribiose, umfassend einen Schritt zur glykosidischen Bindung zwischen einem Glycosyl-Donor und einem -Akzeptor, **dadurch gekennzeichnet, dass**:
• der Glykosyl-Donor in Pyranosidform vorliegt und der Formel (II) entspricht 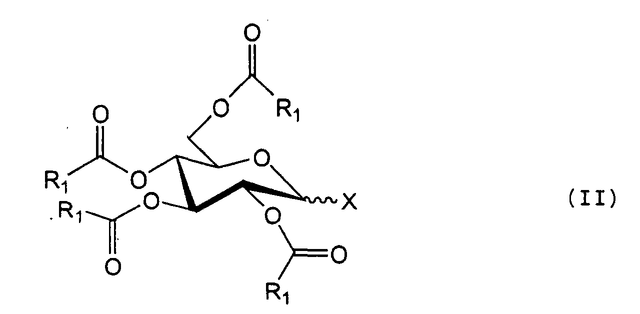
bei der R₁ darstellt:
- einen Alkyl- oder Halogenalkyrest mit 1 bis 6 Kohlenstoffatomen;
- einen nicht substituierten oder durch eine oder mehrere Gruppen substituierten Arylrest, ausgewählt aus einem Halogenatom, einem Alkoxyrest mit 1 bis 6 Kohlenstoffatomen oder einer Nitrogruppe;
X eine nucleofuge Abgangsgruppe dargestellt, ausgewählt aus:
- einer Gruppe mit der Formel S(O)ₙR', in der R' ein Alkylrest mit 1 bis 6 Kohlenstoffatomen, einen nicht substituierten oder mit einer Alkoxygruppe mit 1 bis 6 Kohlenstoffatomen, einer Nitro- oder Acetamidgruppe substituiert ist, und n eine ganze Zahl ist, die gleich 0 oder 1 ist;
- einer Trichloracetimidatgruppe;
• der Glykosyl-Akzeptor in Furanosidform vorliegt und der Formel (III) entspricht 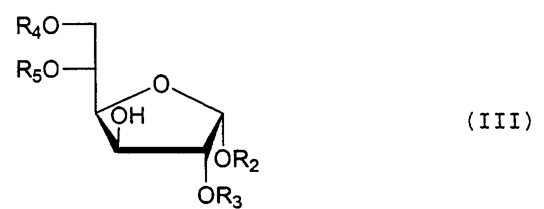 in der:
R₂ und R₃ zusammen einen Methylidyl- (Méthylidyle), Ethylidyl-, Trichlorethylidyl-, Isopropylidyl-, Hexafluorisopropylidyl-, Cyclopentylidyl-, Cyclohexylidyl-, Cycloheptylidyl-, Butylidyl-, 1-tert-Butylethylidyl-, 1-Phenylethylidyl-, Benzylidyl-, Methoxybenzylidyl-, 1-Phenylbenzylidyl-Rest bilden;
R₄ und R₅ zusammen einen Methylidyl-, Ethylidyl-, Trichlorethylidyl-, Isopropylidyl-, Hexafluorisopropylidyl-, Cyclopentylidyl-, Cyclohexylidyl-, Cycloheptylidyl-, Butylidyl-, 1-tert-Butylethylidyl-, 1-Phenylethylidyl-, Benzylidyl-, Methoxybenzylidyl-, 1-Phenylbenzylidyl-Rest bilden, oder unabhängig einen Benzyl-, Acetyl-, Benzoyl-, Chlorbenzoyl-, Methoxybenzoyl-, Nitrobenzoyl-, Allyl-, Chlorbenzyl-, Methoxybenzyloder Nitrobenzyl-Rest darstellen;
• der Bindungsschritt in Lösung in einem wasserfreien, organischen Lösungsmittel bei einer Temperatur zwischen -80°C und 40°C für eine Dauer von 1 Minute bis 8 Stunden in Gegenwart eines geeigneten Initiators durchgeführt wird, der ausgewählt wird aus:
- einer Haloniumionen-Quelle, die an eine Lewissäure assoziiert ist oder nicht, oder an ein Salz einer starken Säure, in dem Fall, dass X eine S(O)ₙR'-Gruppe darstellt, wie oben beschrieben, in der n gleich 0 ist;
- einer an ein Amin assoziierten Lewissäure, in dem Fall, dass X eine S(O)ₙR'-Gruppe, wie oben beschrieben darstellt, in der n gleich 1 ist;
- einer Brönstedtsäure oder einer Lewissäure, in dem Fall, dass X eine Trichloracetimidatgruppe darstellt;
• das dadurch erhaltene, neutralisierte und gereinigte Reaktionsprodukt, das einer Behandlung zur Entfernung der Schutzgruppen unterzogen wurde, um nach der Reinigung zur Laminaribiose zu führen.

2. Verfahren gemäß Anspruch 1, **dadurch gekennzeichnet, dass**
• der Glykosyl-Donor der oben erwähnten Formel (II) entspricht, in der
R₁ einen Rest darstellt, der aus der Gruppe ausgewählt ist, gebildet aus Methyl-, Chlormethyl-, Trifluormethyl-, tert-Butyl-, Phenyl-, Chlorphenyl-, Methoxyphenyl- und Nitrophenyl-Resten;
X einen Rest darstellt, der aus der Gruppe ausgewählt wird, die aus Thiomethyl-, Thioethyl-, Thiopropyl-, Thiophenyl-, Thionitrophenyl-, Thiopyridyl-Resten gebildet wird.

3. Verfahren gemäß den Ansprüchen 1 oder 2, **dadurch gekennzeichnet, dass** der oben erwähnte Initiator ausgewählt wird aus:
- dem N-Bromsuccinimid oder dem N-Iodsuccinimid, an eine Lewissäure assoziiert oder nicht, ausgewählt aus Eisenchlorid, Kupferditriflat, Zinnditriflat, Trifluorboretherat, Zinn- oder Zirkoniumtetrachlorid, Methyltriflat, Trimethyl- (oder Triethyl-)silyltriflat, Silbertriflat, Kadmiumditriflat, Kobaltditriflat, Nickelditriflat, Zinkditriflat, Wismuthtritriflat, Eisentritriflat, Galliumtritriflat, oder an ein Salz einer starkes Säure wie das Tetrabutylammoniumtriflat, in dem Fall, dass X eine S(O)ₙR'-Gruppe, wie oben definiert darstellt, in der n gleich 0 ist,
- einer Lewissäure, ausgewählt aus Trifluormethansulfonsäureanhydrid, Eisenchlorid, Kupferditriflat, Zinnditriflate, Trifluorboretherat, Zinn- oder Zirkoniumtetrachlorid, Methyltriflat, Trimethyl- (oder Triethyl-)silyltriflat, Silbertriflat, Kadmiumditriflat, Kobaltditriflat, Nickelditriflat, Zinkditriflat, Wismuthtritriflat, Eisentritriflat, Galliumtritriflat, assoziiert an ein Amin wie insbesondere das Di-tert-butylmethylpyridin, in dem Fall, dass X eine S(O)ₙR'-Gruppe darstellt, wie oben definiert, in der n gleich 1 ist,
- einer Brönstedtsäure, wie insbesondere Trifluormethansulfonsäure oder para-Toluolschwefelsäure oder einer Lewissäure, ausgewählt aus Trifluormethansulfonsäureanhydrid, Eisenchlorid, Kupferditriflat, Zinnditriflat, Trifluorboretherat, Zinn- oder Zirkoniumtetrachlorid, Methyltriflat, Trimethyl- (oder Triethyl-)silyltriflat, Silbertriflat, Kadmiumditriflat, Kobaltditriflat, Nickelditriflat, Zinkditriflat, Wismuthtritriflat, Eisentritriflat, Galliumtritriflat, in dem Fall, dass X eine Trichloracetimidatgruppe darstellt.

4. Verfahren gemäß Anspruch 2 oder 3, **dadurch gekennzeichnet, dass**
• der Glykosyl-Donor der oben genannten Formel (II) entspricht, in der:
R₁ einen Phenylrest darstellt; und
X einen S(O)ₙR'-Rest darstellt, in dem n gleich 0 ist und R' einen Ethyl- oder Phenylrest darstellt;
• der Glykosyl-Akzeptor der oben erwähnten Formel (III) entspricht, in der R₂, R₃ und R₄, R₅ zusammen einen Cyclohexylidyl- oder Isopropylidyl-Rest bilden.

5. Verfahren gemäß Patentanspruch 4, **dadurch gekennzeichnet, dass**
• der oben erwähnte Initiator eine Mischung aus N-Iodsuccinimid und Zinnditriflat ist, bevorzugt in den Verhältnissen zwischen 1 : 0,5 und 1 : 0,005.

6. Verfahren gemäß einem der Patentansprüche 1 bis 5, **dadurch gekennzeichnet, dass** die oben erwähnte Behandlung zur Entfernung von Schutzgruppen folgendes umfaßt:
a) die Abspaltung der Acetalgruppen, die vom Glykosyl-Akzeptor stammen durch eine Säurebehandlung in wässrigem oder wässrig-organischem Medium, oder in Gegenwart eines sauren Harzes;
b) die Reinigung des so erhaltenen Produkts;
c) die Umesterung oder die Hydrolyse des in Schritt b) erhaltenen Produkts;
d) die Reinigung des auf diese Weise erhaltenen Produkts.

7. Verfahren gemäß Patentanspruch 6, **dadurch gekennzeichnet, dass** der oben erwähnte Schritt a) durch die Umsetzung des neutralisierten und gereinigten Bindungsprodukts in einer Mischung durchgeführt wird, bevorzugt in gleichem Volumen, aus Trifluoressigsäure und Wasser bei einer Temperatur zwischen 10 und 70°C während einer Dauer von 1 Stunde bis 10 Tagen, und dass der oben erwähnte Reinigungsschritt b), bevorzugt in Methanol, durch fraktioniertes Kristallisieren durchgeführt wird.

8. Verfahren gemäß den Patentansprüchen 6 oder 7, **dadurch gekennzeichnet, dass** der oben erwähnte Umesterungsschritt in einem alkoholischen Lösungsmittel wie Methanol oder Ethanol, in Gegenwart einer katalytischen Menge an Natrium, oder Natrium- oder Kaliummethylat oder -ethylat während einer Dauer von 1 Minute bis 10 Tagen durchgeführt wird, und dass der oben erwähnte Reinigungsschritt d) folgendes umfaßt:
d1) die Neutralisierung des in Schritt c) erhaltenen Produkts,
d2) die Entfernung des gebildeten Benzoesäureesters, entweder durch azeotropes Verdampfen mit Wasser oder durch Extraktion mit einem organischen Lösungsmittel,
d3) die Konzentrierung der restlichen wässrigen Phase unter reduziertem Druck,
d4) schließlich, die Lyophilisierung oder die Kristallisierung der so erhalten Laminaribiose in einer wässrig-alkoholischen Mischung.

9. Verfahren gemäß einem der vorhergehenden Patentansprüche, **dadurch gekennzeichnet, dass** der oben erwähnte Bindungsschritt in Lösung in Dichlormethan, 1,2-Dichlorethan oder Toluol durchgeführt wird, bevorzugt in Gegenwart eines molekularen Siebs bei einer Temperatur zwischen -30°C und 30°C während einer Dauer von 1 Minute bis 6 Stunden, bevorzugt bei 10°C für 30 Minuten.

10. Zwischenprodukte der Laminaribiosesynthese, **dadurch gekennzeichnet, dass** sie aus den folgenden Verbindungen ausgewählt werden

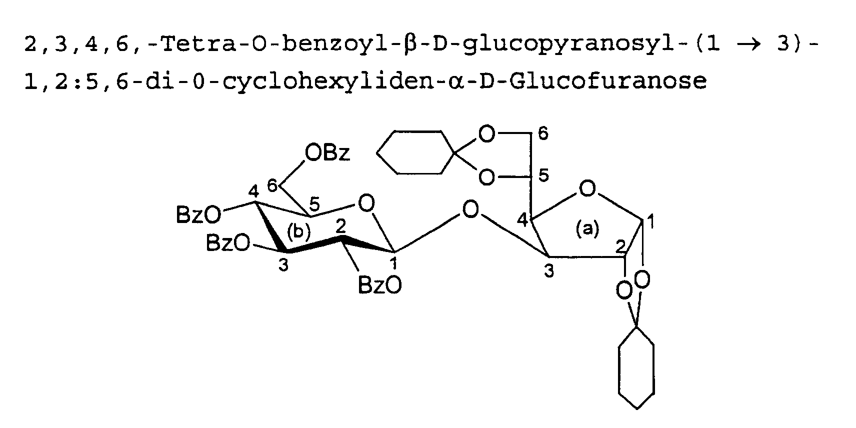

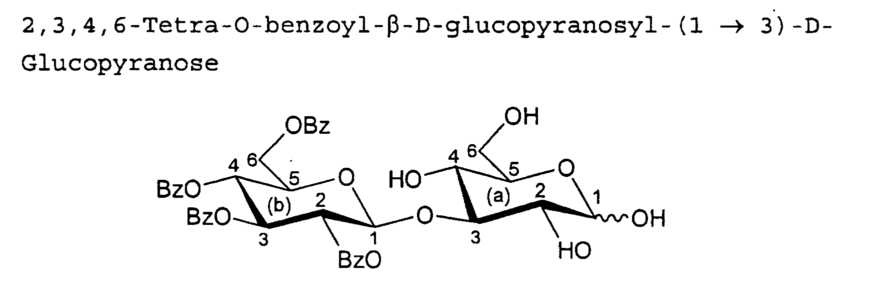

## Claims

1. A method of preparing laminaribiose comprising a step of glycosidic coupling between a glycosyl donor and a glycosyl acceptor, **characterised in that** :
• the glycosyl donor is in pyranose form and is of formula (II) : 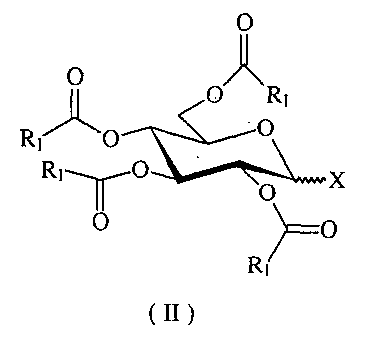 in which :
R₁ represents
- an alkyl or haloalkyl radical having 1 to 6 carbon atoms ;
- an aryl radical which is non-substituted or substituted with one or more groups selected from a halogen atom, an alkoxy radical having 1 to 6 carbon atoms or a nitro group ;
X represents an electrophilic leaving group selected from:
- a group of formula S(O)ₙR' in which R' represents an alkyl radical having I to 6 carbon atoms, an aryl radical which is non-substituted or substituted with an alkoxy group having 1 to 6 carbon atoms, a nitro or acetamide group, and n is an integer equal to 0 or 1; or
- a trichloroacetimidate group ;
• the glycosyl acceptor is in furanose form and is of formula (III) 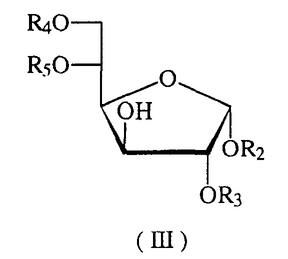 in which :
R₂ and R₃ together form a methylidyl, ethylidyl, trichloroethylidyl, isopropylidyl, hexafluoroisopropylidyl, cyclopentylidyl, cyclohexylidyl, cycloheptylidyl, butylidyl, 1-tert-butylethylidyl, 1-phenylethylidyl, benzylidyl, methoxybenzylidyl, or 1-phenylbenzylidyl radical ; and
R₄ and R₅ together form a methylidyl, ethylidyl, trichloroethylidyl, isopropylidyl, hexafluoroisopropylidyl, cyclopentylidyl, cyclohexylidyl, cycloheptylidyl, butylidyl, 1-tert-butylethylidyl, 1-phenylethylidyl, benzylidyl, methoxybenzylidyl, or 1-phenylbenzylidyl radical, or independently represent a benzyl, acetyl, benzoyl, chlorobenzoyl, methoxybenzoyl, nitrobenzoyl, allyl, chlorobenzyl, methoxybenzyl or nitrobenzyl radical;
• said coupling step is carried out in solution in an anhydrous organic solvent, at a temperature between -80°C and 40°C, for a period of 1 minute to 8 hours, in the presence of a suitable promoter selected from :
- a source of halonium ions, combined or not with a Lewis acid or a salt of a strong acid, in the case in which X represents an S(O)ₙR' group as defined above in which n is equal to 0 ;
- a Lewis acid combined with an amine, in the case in which X represents an S(O)ₙR' group as defined above in which n is equal to 1 ; or
- a Bronsted acid or a Lewis acid, in the case in which X represents a trichloroacetimidate group ; and
• the reaction product thus obtained, neutralised and purified, being subjected to a deprotection treatment to give, after purification, laminaribiose.

2. The method according to claim 1, **characterised in that** :
• the glycosyl donor is of formula (II) mentioned above in which :
R₁ represents a radical selected from the group consisting of methyl, chloromethyl, trifluoromethyl, tert-butyl, phenyl, chlorophenyl, methoxyphenyl and nitrophenyl radicals ; and
X represents a radical selected from the group consisting of thiomethyl, thioethyl, thiopropyl, thiophenyl, thionitrophenyl and thiopyridyl radicals.

3. The method according to claim 1 or 2, **characterised in that** the promoter mentioned above is selected from :
- N-bromosuccinimide or N-iodosuccinimide, combined or not with a Lewis acid selected from ferric chloride, copper ditriflate, tin ditriflate, boron trifluoride dietherate, tin or zirconium tetrachloride, methyl triflate, trimethyl- (or triethyl-) silyl triflate, silver triflate, cadmium ditriflate, cobalt ditriflate, nickel ditriflate, zinc ditriflate, bismuth tritriflate, iron tritriflate, gallium tritriflate, or with a salt of a strong acid such as tetrabutylammonium triflate, in the case in which X represents an S(O)ₙR' group as defined above in which n is equal to O,
- a Lewis acid selected from triflic anhydride, ferric chloride, copper ditriflate, tin ditriflate, boron trifluoride dietherate, tin or zirconium tetrachloride, methyl triflate, trimethyl- (or triethyl-) silyl triflate, silver triflate, cadmium ditriflate, cobalt ditriflate, nickel ditriflate, zinc ditriflate, bismuth tritriflate, iron tritriflate, gallium tritriflate, combined with an amine such as di-tert-butylmethylpyridine in particular, in the case in which X represents an S(O)ₙR' group as defined above in which n is equal to 1, and
- a Bronsted acid particularly such as triflic acid or para-toluenesulphonic acid or a Lewis acid selected from triflic anhydride, ferric chloride, copper ditriflate, tin ditriflate, boron trifluoride dietherate, tin or zirconium tetrachloride, methyl triflate, trimethyl- (or triethyl-) silyl triflate, silver triflate, cadmium ditriflate, cobalt ditriflate, nickel ditriflate, zinc ditriflate, bismuth tritriflate, iron tritriflate, gallium tritriflate, in the case in which X represents a trichloroacetimidate group.

4. The method according to claim 2 or 3, **characterised in that**:
• the glycosyl donor is of formula (II) mentioned above in which :
R₁ represents a phenyl radical; and
X represents an S(O)ₙR' radical in which n is equal to O and R' represents an ethyl or phenyl radical;
• the glycosyl acceptor is of formula (III) mentioned above in which :
R₂, R₃ and R₄, R₅ together form a cyclohexylidyl or isopropylidyl radical.

5. The method according to claim 4, **characterised in that** :
• the promoter mentioned above is a mixture of N-iodosuccinimide and of tin ditriflate, preferably in proportions between 1:0.5 and 1:0.005.

6. The method according to one of claims 1 to 5, **characterised in that** the deprotection treatment mentioned above comprises :
a) cleaving the acetal groups originating from the glycosyl acceptor by an acidic treatment in an aqueous or hydro-organic medium, or in the presence of an acidic resin ;
b) purifying the product thus obtained ;
c) transesterifying or hydrolysing the product obtained in step b) ; and
d) purifying the product thus obtained.

7. The method according to claim 6, **characterised in that** step a) mentioned above is carried out by allowing the neutralised and purified coupling product to react in a mixture, preferably of equal volumes of trifluoroacetic acid and water, at a temperature between 10 and 70 °C for a period of 1 hour to 10 days, and **in that** the purification step b) mentioned above is carried out by fractional crystallisation, preferably in methanol.

8. The method according to claim 6 or 7, **characterised in that** the transesterification step mentioned above is carried out in an alcoholic solvent such as methanol or ethanol, in the presence of a catalytic amount of sodium or of sodium or potassium methoxide or ethoxide for a period of 1 minute to 10 days, and **in that** the purification step d) mentioned above comprises :
d1) neutralising the product obtained in step c),
d2) removing the benzoic ester formed, either by azeotropic evaporation with water, or by extraction with an organic solvent,
d3) concentrating under reduced pressure the residual aqueous phase, and
d4) optionally, lyophilising or crystallising the laminaribiose thus obtained in a hydro-alcoholic mixture.

9. The method according to one of the preceding claims, **characterised in that** the coupling step mentioned above is carried out in solution in dichloromethane, 1,2-dichloroethane or toluene, preferably in the presence of molecular sieves, at a temperature between - 30°C and 30°C, for a period of 1 minute to 6 hours, preferably at 10°C for 30 minutes.

10. Laminaribiose synthesis intermediates, **characterised in that** they are selected from the following compounds : 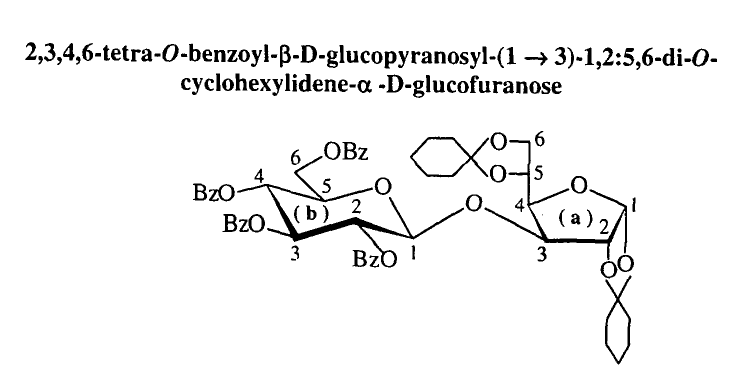 and
